# EUROPEAN PATENT SPECIFICATION

(11) **EP 0 730 735 B1**
(45) Date of publication and mention of the grant of the patent: **02.04.2003**
(21) Application number: 95903574.2
(22) Date of filing: 14.11.1994
(51) Int. Cl.: G01N 31/22

(54) **GLUCOSE CALIBRATOR AND CONTROL MATERIAL FOR TEST STRIPS**
GLUCOSEKALIBRATOR UND KONTROLLMATERIAL FÜR TESTSTREIFEN
PREPARATION D'ETALONNAGE ET DE CONTROLE POUR BANDELETTES D'ESSAI POUR LA DETERMINATION DU GLUCOSE

(30) Priority: 12.11.1993 US 152541
(43) Date of publication of application: 11.09.1996
(73) Proprietor: Roche Diagnostics Corporation, Indianapolis, IN 46250 (US)
(72) Inventor: LEE, Debra, McDonald, Indianapolis, IN 46205 (US); BURKE, David William, Carmel, IN 46032 (US); TABB, David, Lee, Indianapolis, IN 46140 (US)
(74) Representative: Jung, Michael, Dr.
(86) International application number: US9413416
(87) International publication number: WO95013535

(56) References cited:
- EP-A- 0 266 216
- WO-A-93/21928
- WO-A-94/14058
- GB-A- 2 002 516
- JP-A- 3 112 908
- JP-A- 62 000 412
- US-A- 3 876 375
- US-A- 3 920 580
- US-A- 4 729 959
- US-A- 5 028 542
- US-A- 5 563 233
- DATABASE WPI Section Ch, Week 198528 Derwent Publications Ltd., London, GB; Class A96, AN 1985-168124 XP002152355 & JP 60 097043 A (SHISEIDO CO LTD), 30 May 1985 (1985-05-30)
- DATABASE WPI Section Ch, Week 198636 Derwent Publications Ltd., London, GB; Class D21, AN 1986-236766 XP002152356 & JP 61 167608 A (SHISEIDO CO LTD), 29 July 1986 (1986-07-29)

## Description

### BACKGROUND

The present invention relates to calibrator and control material useful in calibrating and validating testing devices such as test strips and dipsticks. More particularly, the present invention relates to a non-serum based, aqueous glucose calibrator and control material and to a method for making said calibrator and control material.

The field of clinical chemistry and clinical analysis is concerned, *inter alia,* with the determination and quantification of various substances in body fluids. Many examples of the substances which are determined can be given, and these include cholesterol, urea, cations, and glucose. These examples of analytes, as well as others, are assayed in diverse body fluids such as urine and blood.

The monitoring of the level of glucose in blood is important to the management of diabetes. The level of glucose in the blood is controlled by the amount of carbohydrate ingested and by insulin. Too much insulin lowers the glucose level, and too little will result in an abnormally high level of glucose. Both circumstances lead to serious health problems for the diabetic.

Most of the glucose testing done outside of the hospital laboratory is done in non-laboratory settings such as nurses' stations, physicians' offices and at home. Testing is frequently done by measuring the amount of glucose in urine. As the level of glucose rises in the blood, it exceeds the ability of the kidney to reabsorb it, and glucose is excreted into the urine.

Although measurement of glucose in urine is useful, measurement of glucose in blood provides a more accurate reflection of the condition of the subject. Urine glucose does not accurately reflect the level of glucose in the blood since the level of glucose in urine is determined by the level of glucose in the blood and the ability of the kidney to reabsorb the glucose.
Therefore, the urine sample cannot tell the diabetic how low his glucose level is.

Dry reagent test strips, sometimes referred to as dipsticks, are widely used for detecting glucose in urine and blood. These devices are characterized by their simplicity of use. In general, such test strips comprise plastic strips provided at one end thereof with an absorbent paper portion which has been impregnated with reagents such as an enzyme system and a color indicator compound which produces or changes color to form a detectable signal when the test strip is contacted with the analyte being determined. This change in color can be measured by comparing the color formed on the strip with a standard color chart calibrated to various glucose concentrations. More recently, however, to more accurately control the level of glucose in blood, instruments have been developed which measure the color change in a reflectance photometer and thereby produce quantitative results. Examples of reaction systems which measure glucose using reflectance measurements include oxidative reactions, such as the glucose oxidase/peroxidase method, and reductive reactions, such as the glucose oxidase/ferricyanide method. The latter method is described in detail in Freitag, U.S. Pat. No. 4,929,545. Instruments have also been developed which determine glucose by means of electrochemical methods in which a change in current is measured. An example of this technology is the amperometric, biosensor method described in PCT Application No. PCT/US90/07374, published as WO91/09139.

It will be understood that clinical analysis of the type described herein requires that any testing system be extremely accurate. In particular, when automated systems and instruments are used, it is essential to ensure that the elements of the analysis are reliable and that the measurement taken is valid. It is for this purpose that calibrator and control reagents are used.

Westgard and Klee, in Textbook of Clinical Chemistry, N.W. Tietz, Ed., 1986, p. 430, define "reference material" as "a material or substance one or more properties of which are sufficiently well established to be used for the calibration of an apparatus or for the verification of a measurement method." "Calibration and test material" is defined as "a reference material or solution with which the test sample is compared in order to determine the concentration of analytes or other quantities."

The Westgard and Klee reference defines "control material" as "a specimen, or solution, which is analyzed solely for quality control purposes and is not used for calibration purposes." This standard reference work goes on to describe some of the requisites of control materials as follows: "They need to be stable materials, available in aliquots or vials, that can be analyzed periodically over a long time. There should be little vial-to-vial variation so that differences between repeated measurements can be attributed to the analytical method alone."

The above-cited reference, at page 433, discusses how the matrix of the control material should be the same as the material being analyzed. To that end, modified human serum is discussed as one type of control material. Indeed, the art now recognizes the term "control serum" as referring to control material based upon serum. This terminology will be used herein and is different from the term "control reagent," which, as used hereafter, refers to a control material which is not based upon, and which does not contain, serum of any type.

As has been pointed out above, one of the criteria which control, and also calibration, materials have to satisfy is stability. Control materials based upon serum, however, are inherently unstable due to the various components contained therein. Further, sera will vary from source to source, so uniformity from lot to lot cannot be guaranteed. Thus, it is sometimes desirable to have a calibrator or control material based upon a non-serum or serum-free medium.

An example of a serum-free control medium, or "control reagent" as used herein, is described in U.S. Pat. No. 4,729,959, issued to Ryan, which is directed to "a stable glucose reference control." This control contains glucose in a range of from about 40 to 500 mg/dl, together with fixed red blood cells, in an aqueous suspension. The range of glucose concentrations given are sufficient to cover just about all ranges of glucose found in, e.g., blood.

The Ryan '959 patent points to a problem with aqueous control reagents at column 1, lines 50-55. Briefly, erythrocytes impart a degree of viscosity to blood which is absent in water based systems. This problem was also recognized in U.S. Pat. No. 3,920,580 issued to Mast. This patent teaches that aqueous solutions had not been consistent, and that a lack of reproducibility was observed when dry reagent strips were used with such materials. Mast teaches that suitable reagents could be prepared by using an antidiffusing agent in combination with glucose and water. The antidiffusing agents taught by Mast include polyvinylpyrrolidone, polyvinyl alcohol, polyethylene glycol, dextran, and bovine serum albumin. Beneficial amounts are taught to be between about 3 and 35 percent of antidiffusing agent. The control solution may also include adjuvants to obtain a particular color or physical appearance, which include colored latex particles and water-insoluble lake dyes.

Maurukas, in U.S. Pat. No. 3,876,375, describes a reference control which is stable in the liquid state at temperatures ranging from -20°C to ambient room temperatures. Maurukas' compositions are prepared by removing from 20 to 40% of the water from an aqueous biological material and replacing it with substantially the same concentration of an alkylene polyol containing from 2 to 5 carbon atoms. Suggested alkylene polyols are ethylene glycol, propylene glycol, butylene glycol, pentanediol and glycerol. The compositions described are said to be suitable for serum analysis using sequential multichannel automated analyzers. No mention is made as to the possible suitability of the compositions with test strips for measuring glucose using whole blood samples.

Kennamer *et al*., in U.S. Pat. No. 5,028,542 describe a non-serum based, glucose measurement control reagent in which the viscosity agent polystyrene sulfonate is used.

JP-A 61 167608 describes cosmetic compositions comprising water, glucose and dipropylene glycol. WO 94/14058, which represents prior art within the meaning of Art. 54(3) EPC, describes an aqueous control reagent containing glucose, an antioxidant (e.g. TBHQ), a preservative, ionic salts, and a buffer.

It has now been found that a suitable glucose calibrator and control reagent can be formed without using any of the materials referred to in the prior art as required ingredients. Rather, by combining a soluble dihydroxy alcohol with a predetermined amount of glucose and water, along with additional optional materials, a suitable glucose calibrator or control reagent can be made.

### SUMMARY OF THE INVENTION

The present invention is a non-serum based glucose calibrator or control reagent which comprises a predetermined, known amount of glucose, water, and dipropylene glycol. A preferred concentration for the latter is between about 20 to 30 percent by weight of the reagent composition. It was found, quite unexpectedly, that the composition of the present invention is useful in calibrating and validating testing devices such as test strips for the measurement of glucose. Additional materials such as a buffer, a preservative, a biocide, an ionic salt, or a surfactant, either alone or in various additive combinations, may be mixed with the three required components. Another aspect of the present invention is a method for making the calibrator or control reagent by mixing the glucose, water, and dipropylene glycol together.

Essential to the invention are a predetermined amount of glucose, water, and dipropylene glycol. The water is used, of course, to create a reagent solution in which the other components are dissolved. By "predetermined" is meant that, prior to formulation of the actual reagent, a concentration of glucose has been selected. This concentration may vary, as those skilled in the art will recognize. As has been mentioned above, the art recognized, *e.g.*, a range of from 40 to 500 mg/dl for control material, but one may envision broader ranges, *e.g*., from about 10 to 700 mg/dl, when the material is to be used for calibration purposes.

The essential features of the invention, when the reagent is in the form of a solution, are the solvent (water), the predetermined amoumt of glucose, and dipropylene glycol. The latter may be present in, *e.g.,* a range of about 20 to about 30 percent by weight of the reagent. The weight percent of dipropylene glycol will be determined by criteria such as the final reagent viscosity desired and the desired diffusion or permeability characteristics of the calibrator or control with the particular testing device with which it is to be used. Of course, the particular amount of dipropylene glycol selected should also be one which does not adversely interfere with the determination of glucose or have a negative effect on reagent stability. It is not necessary that the control material have the same viscosity as whole blood; however, it is desirable that the permeability of the material, i.e., the diffusion rate of the glucose analyte, through the reagent matrix of the test strip approximate that of whole blood.

Optional additional components of the calibrator or control reagent include typical additives such as buffers, preservatives, surfactants, and ionic salts. With respect to buffers, some preferred species are succinate, HEPES (4-[2-hydroxyethyl-1-piperazine] ethane sulfonic acid) , CHES (2-[N-cyclohexylamino] ethane sulfonic acid), MOPS (3-[N-morpholino] propane sulfonic acid), MEPS (2-[N-morpholino] ethane sulfonic acid), and CAPS (3-[cyclohexylamino-1-1-propane] sulfonic acid) buffers. Preferred preservatives or biocides include imidazolidinyl urea, available under the trade name GERMALL 115 (GAF Chemicals Corp.), methylparaben (methyl-p-hydroxybenzoate) or methanol (((2-(dihydro-5-methyl-3(2H)-oxazoylyl)-1-methylethoxy)methoxy)methoxy), available as COSAN 145 (Cosan Chemical Corp.), phenoxyethanol and gentamycin sulfate, both individually and in combination.

It may also be desirable to include a colored or colorable substance in the reagent mixture. This can be desirable because body fluid samples frequently possess a particular color as one of their properties. As the control reagent is being used to calibrate per a body fluid sample, it can be useful to calibrate against conditions as similar to the tested fluid as possible, including color.

### DESCRIPTION OF PREFERRED EMBODIMENTS

### Example 1

A preferred formulation of the calibrator and control reagent of the present invention was prepared having the following composition:

| | |
|---|---|
| HEPES, Na salt | 30 mM |
| HEPES, free acid | 30 mM |
| CaCl2 | 130 mM |
| NaCl | 120 mM |
| GERMALL 115 | 0.3 % |
| Methylparaben | 0.3 % |
| Water | 77.5 % |
| Dipropylene glycol | 22.5 % |

This reagent was adjusted with NaOH to have a final pH of 7.5. Following preparation, the reagent was divided into aliquots and spiked with predetermined amounts of glucose. Theoretical or target glucose levels selected were 10, 20, 40, 60, 80, 100, 130, 180, 300, 550 and 700 mg/dl. Each of the calibrator/control mixtures was then divided into vials, and duplicates were stored under a variety of temperature conditions.

### Example 2

Each of the calibrator and control mixtures described in Example 1 was then tested for its efficacy after 1 week of storage at 25°C. As explained above, one of the most important features of a control reagent is its consistency, meaning that values obtained using it should be fairly uniform from run to run.

With this in mind, each of the control materials stored at 25°C was applied to test strips containing the glucose determination system described in PCT/US90/07374 (WO 91/09139). Briefly, this publication describes the determination of glucose using an amperometric biosensor system.

Twelve replicates of each calibration/control mixture were measured, and the mean current readings at 10 seconds, standard deviations, arid coefficients of variation were calculated. Reference glucose values for the samples were determined using hexokinase methodology and an HITACHI 705 (Boehringer Mannheim Corp., Indianapolis, IN) analyzer. The results obtained are shown in Table 1 below.

**Table 1**

| HK Glucose (mg/dl) | Mean (µA) | Std. Dev. | Coeff. of Variation |
|---|---|---|---|
| 11.0 | 1.30 | 0.06 | 4.75 |
| 41.5 | 3.81 | 0.07 | 1.84 |
| 63.0 | 5.85 | 0.09 | 1.60 |
| 85.0 | 7.90 | 0.10 | 1.31 |
| 106.0 | 9.98 | 0.12 | 1.17 |
| 137.5 | 13.18 | 0.21 | 1.63 |
| 191.0 | 18.30 | 0.16 | 0.87 |
| 316.5 | 30.29 | 0.92 | 3.04 |
| 573.5 | 52.40 | 2.31 | 4.41 |
| 711.5 | 64.05 | 3.81 | 5.96 |

These results show a level of consistency well within that required of a calibration and control reagent, as is indicated by the comparative standard deviation values and coefficients of variation reported for each set of tests.

## Claims

1. A calibrator or control reagent for glucose determination comprising a mixture of a predetermined amount of glucose, water, and dipropylene glycol.

2. The calibrator or control reagent of claim 1, wherein said dipropylene glycol is present in an amount ranging from 20 to 30 percent by weight of said calibrator or control reagent.

3. The calibrator or control reagent of claim 1, further comprising a buffer.

4. The calibrator or control reagent of claim 1, further comprising a preservative.

5. The calibrator or control reagent of claim 1, further comprising an ionic salt.

6. The calibrator or control reagent of claim 1, further comprising a buffer, a preservative, and an ionic salt.

7. A process for making a calibrator or control reagent for glucose determination comprising combining a predetermined amount of glucose, water, and dipropylene glycol.

8. The process of claim 7, wherein said dipropylene glycol is present in an amount ranging from 20 to 30 percent by weight of said calibrator or control reagent.

9. The process of claim 7, further comprising combining a material selected from the group consisting of a buffer, a preservative, and an inorganic salt with said predetermined amount of glucose, water, and dipropylene glycol.

## Patentansprüche

1. Kalibrator oder Kontrollreagenz für die Glucosebestimmung, der bzw. das eine Mischung aus einer vorbestimmten Menge Glucose, Wasser und Dipropylenglykol umfasst.

2. Kalibrator oder Kontrollreagenz nach Anspruch 1, worin das Dipropylenglykol in einer Menge im Bereich von 20 bis 30 Gew.-% des Kalibrators oder Kontrollreagenz vorhanden ist.

3. Kalibrator oder Kontrollreagenz nach Anspruch 1, der bzw. das weiterhin einen Puffer umfasst.

4. Kalibrator oder Kontrollreagenz nach Anspruch 1, der bzw. das weiterhin ein Konservierungsmittel umfasst.

5. Kalibrator oder Kontrollreagenz nach Anspruch 1, der bzw. das weiterhin ein ionisches Salz umfasst.

6. Kalibrator oder Kontrollreagenz nach Anspruch 1, der bzw. das weiterhin einen Puffer, ein Konservierungsmittel und ein ionisches Salz umfasst.

7. Verfahren zur Herstellung eines Kalibrators oder Kontrollreagenz für die Glucosebestimmung, wobei eine vorbestimmte Menge Glucose, Wasser und Dipropylenglykol vereint wird.

8. Verfahren nach Anspruch 7, worin das Dipropylenglykol in einer Menge im Bereich von 20 bis 30 Gew.-% des Kalibrators oder Kontrollreagenz vorhanden ist.

9. Verfahren nach Anspruch 7, wobei weiterhin ein Material, das aus der Gruppe gewählt ist, die aus einem Puffer, einem Konservierungsmittel und einem anorganischen Salz besteht, mit der vorbestimmten Menge Glucose, Wasser und Dipropylenglykol vereint wird.

## Revendications

1. Réactif d'étalonnage ou témoin pour la détermination de la teneur en glucose, comprenant un mélange d'une quantité prédéterminée de glucose, d'eau et de dipropylèneglycol.

2. Réactif d'étalonnage ou témoin selon la revendication 1, dans lequel ledit dipropylèneglycol est présent en une quantité qui se situe dans la plage de 20 à 30 % en poids dudit réactif d'étalonnage ou témoin.

3. Réactif d'étalonnage ou témoin selon la revendication 1, comprenant en outre un tampon.

4. Réactif d'étalonnage ou témoin selon la revendication 1, comprenant en outre un agent de conservation.

5. Réactif d'étalonnage ou témoin selon la revendication 1, comprenant en outre un sel ionique.

6. Réactif d'étalonnage ou témoin selon la revendication 1, comprenant en outre un tampon, un agent de conservation et un sel ionique.

7. Procédé pour préparer un réactif d'étalonnage ou témoin pour la détermination de la teneur en glucose comprenant le fait de combiner une quantité prédéterminée de glucose, d'eau et de dipropylèneglycol.

8. Procédé selon la revendication 7, dans lequel ledit dipropylèneglycol est présent en une quantité qui se situe dans la plage de 20 à 30 % en poids dudit réactif d'étalonnage ou témoin.

9. Procédé selon la revendication 7, comprenant en outre le fait de combiner une matière choisie parmi le groupe constitué par un tampon, un agent de conservation et un sel inorganique avec ladite quantité prédéterminée de glucose, d'eau et de dipropylèneglycol.
